Europäisches Patentamt
European Patent Office
Office européen des brevets

(19)

(11) Veröffentlichungsnummer: **0 476 439 A1**

(12) **EUROPÄISCHE PATENTANMELDUNG**

(21) Anmeldenummer: **91114977.1**

(22) Anmeldetag: **05.09.91**

(51) Int. Cl.5: **C07D 253/065**, A61K 31/53, A01N 43/707

(30) Priorität: **18.09.90 DE 4029534**
**19.06.91 DE 4120138**

(43) Veröffentlichungstag der Anmeldung:
**25.03.92 Patentblatt 92/13**

(84) Benannte Vertragsstaaten:
**AT BE CH DE DK ES FR GB GR IT LI LU NL SE**

(71) Anmelder: **BAYER AG**
**W-5090 Leverkusen 1 Bayerwerk(DE)**

(72) Erfinder: **Lindner, Werner, Dr.**
**Märchenstrasse 39**
**W-5000 Köln 80(DE)**
Erfinder: **Haberkorn, Axel, Prof. Dr.**
**Fuhlrottstrasse 99**
**W-5600 Wuppertal 1(DE)**

(54) **Substituierte 1,2,4-Triazindione, Verfahren zu ihrer Herstellung, Zwischenprodukte dafür und ihre Verwendung.**

(57) Die vorliegende Erfindung betrifft;
Neue substituierte 1,2,4-triazindione der allgemeinen Formel I

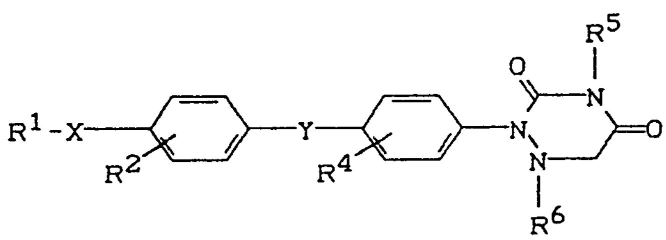

(I)

in welcher

X für O oder S, SO, $SO_2$ steht,

Y für O, S, CO,

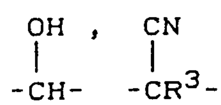

steht,

$R^1$ für $C_{1-4}$-Halogenalkyl steht,

$R^2$ für Wasserstoff, Halogen, $C_{1-4}$-Halogenalkyl steht,

$R^3$ für Wasserstoff, $C_{1-4}$-Alkyl steht,

$R^4$ für einen oder mehrere gleiche oder verschiedene Reste aus der Reihe Wasserstoff, Halogen, Halogenalkyl, $C_{1-4}$-Alkyl steht,

$R^5$ und $R^6$ unabhängig voneinander für Wasserstoff, $C_{1-4}$-Alkyl, Halogenalkyl, Aralkyl, Alkinyl steht,

Verfahren und Zwischenprodukte zur Herstellung der neuen Verbindungen sowie ihre Verwendung zur Bekämp-



Rank Xerox (UK) Business Services

fung parasitärer Protozoen insbesondere von Coccodien sowie Fischparasiten.

Die vorliegende Erfindung betrifft neue substituierte 1,2,4-Triazindione, Verfahren zu ihrer Herstellung, Zwischenprodukte zur Durchführung dieser Verfahren, sowie ihre Verwendung zur Bekämpfung parasitärer Protozoen und insbesondere Coccidien sowie Fischparasiten.

Die Verwendung von substituierten 1,2,4-Triazindionen zur Bekämpfung von Coccidien ist bekannt, Die Wirkung dieser Verbindungen befriedigt jedoch nicht in jedem Fall.

Die vorliegende Erfindung betrifft

1. Neue Verbindungen der Formel (I)

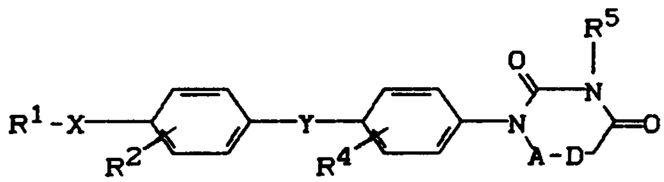

(I)

in welcher

A-D für -N = CH- oder

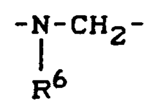

steht

X für O oder S, SO, $SO_2$ steht,

Y für O, S, CO,

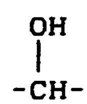 , 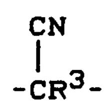

steht,

$R^1$ für $C_{1-4}$-Halogenalkyl steht,

$R^2$ für Wasserstoff, Halogen, $C_{1-4}$-Halogenalkyl steht,

$R^3$ für Wasserstoff, $C_{1-4}$-Alkyl steht,

$R^4$ für einen oder mehrere gleiche oder verschiedene Reste aus der Reihe Wasserstoff, Halogen, Halogenalkyl, $C_{1-4}$-Alkyl steht,

$R^5$ und $R^6$ unabhängig voneinander für Wasserstoff, $C_{1-4}$-Alkyl, Halogenalkyl, Aralkyl, Alkinyl stehen,

2. Verfahren zur Herstellung der neuen Verbindungen der Formel (I) gemäß (1), dadurch gekennzeichnet, daß man in den Fällen in denen A-D für -N = CH-steht,

a) für den Fall, daß $R^5$ für einen anderen Rest als Wasserstoff steht, Verbindungen der Formel (Ia)

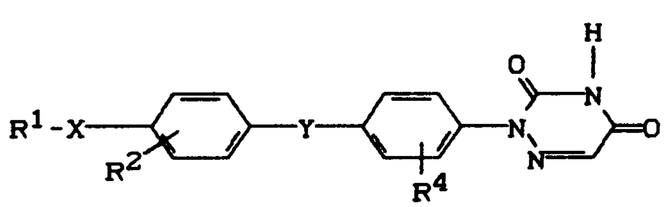

(Ia)

in welcher

Y, X, $R^1$, $R^2$, $R^3$, $R^4$ die in (1) angegebene Bedeutung haben,

mit Verbindungen der Formel (II)

$R^5$-B (II)

in welcher
$R^5$ für gegebenenfalls substituiertes Alkyl, Alkenyl, Alkinyl, Aralkyl steht und
B für Halogen, -O-$SO_2$-Alkyl, -O-$SO_2$-Aryl, -O-$SO_2$-Halogenalkyl steht
umsetzt, oder
b) daß man Verbindungen der Formel (III)

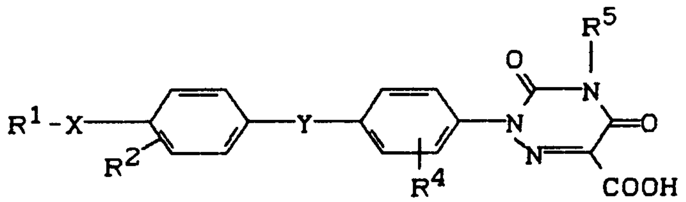

(III)

in welcher
Y, X, $R^1$, $R^2$, $R^3$, $R^4$, $R^5$ die in (1) angegebenen Bedeutungen haben,
durch Erhitzen decarboxyliert, und ferner daß man in den Fällen in denen A-D für

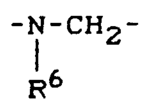

steht,
c) Verbindungen der Formel (Ib)

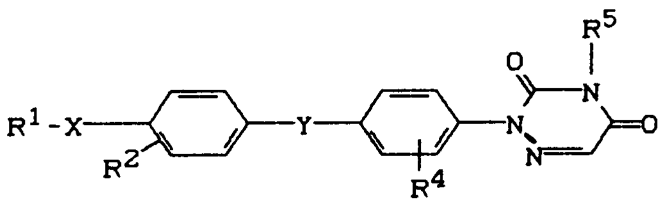

(Ib)

hydriert, oder
d) daß man Verbindungen der Formel

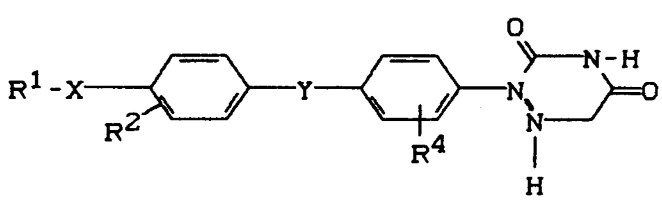

in welcher
Y, X, $R^1$, $R^2$, $R^4$ die oben angegebene Bedeutung haben
mit Verbindungen der Formel II

$R^5$-B (II)

in welcher
$R^5$ für gegebenenfalls substituiertes Alkyl, Alkenyl, Alkinyl, Aralkyl steht und
B für Halogen, -O-$SO_2$-Alkyl, -O-$SO_2$-Aryl, -O-$SO_2$-Halgoenalkyl steht

umsetzt, oder

e) daß man Verbindungen der Formel

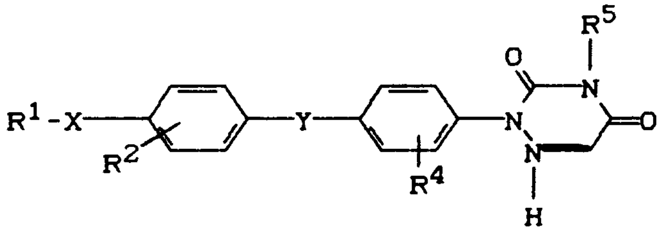

in welcher

Y, X, $R^1$, $R^2$, $R^4$, $R^5$ die oben angegebene Bedeutung haben, $R^5$ aber nicht für Wasserstoff steht, mit Verbindungen der Formel VI

$R^6$-B (VI)

in welcher

$R^6$ und B die oben angegebene Bedeutung haben, umsetzt.

3. Neue Verbindungen der Formel (III)

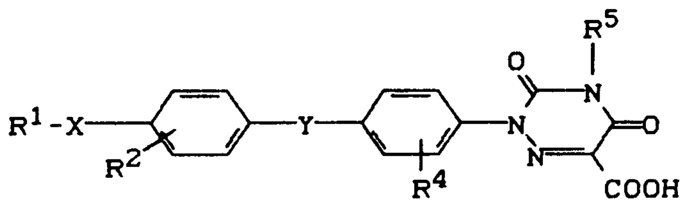

(III)

in welcher

Y, X, $R^1$, $R^2$, $R^3$, $R^4$, $R^5$ die in (1) angegebene Bedeutung haben.

4. Verfahren zur Herstellung der neuen Verbindungen der Formel (III) gemäß (3), dadurch gekennzeichnet, daß man Verbindungen der Formel (IV)

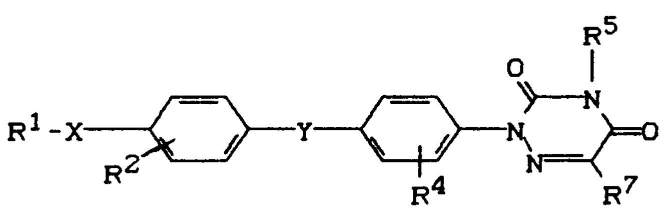

(IV)

in welcher

Y, X, $R^1$, $R^2$, $R^3$, $R^4$, $R^5$ die in (1) angegebene Bedeutung haben,

$R^7$ für CN oder den Rest -CO-N($R^5$)-COOR$^8$ steht,

$R^8$ für Alkyl oder Aryl steht,

in Gegenwart von wäßrigen Mineralsäuren erhitzt.

5. Neue Verbindungen der Formel (IV)

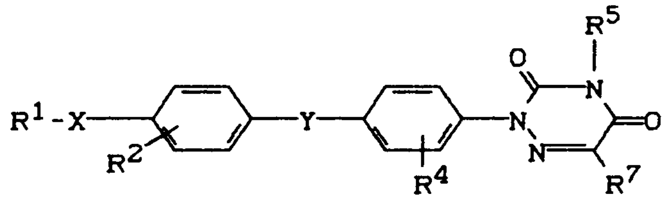

(IV)

in welcher

Y, X, $R^1$, $R^2$, $R^3$, $R^4$, $R^5$, $R^7$ die in (4) angegebene Bedeutung haben.

6. Verfahren zur Herstellung der neuen Verbindungen der Formel (IV) gemäß (5), dadurch gekennzeichnet, daß man Verbindungen der Formel (V)

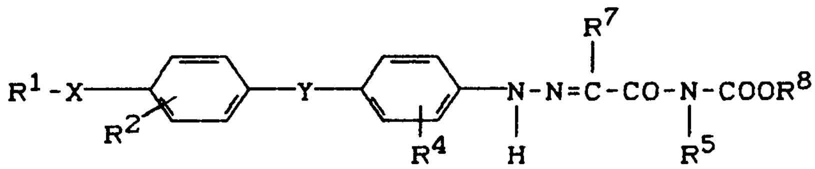

(V)

in welcher

Y, X, $R^1$, $R^2$, $R^3$, $R^4$, $R^5$, $R^7$, $R^8$ die in (5) angegebene Bedeutung haben,

in Gegenwart von Basen erhitzt.

7. Neue Verbindungen der Formel (V)

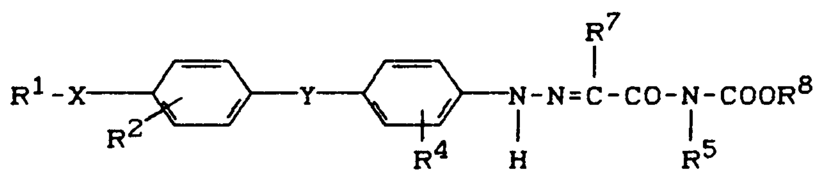

(V)

in welcher

X, $R^1$, $R^2$, $R^3$, $R^4$, $R^5$, $R^7$, $R^8$ die in (5) angegebene Bedeutung haben und $R^7$ zusätzlich für Wasserstoff stehen kann.

8. Verfahren zur Herstellung der neuen Verbindungen der Formel (V), dadurch gekennzeichnet, daß man Verbindungen der Formel (VI)

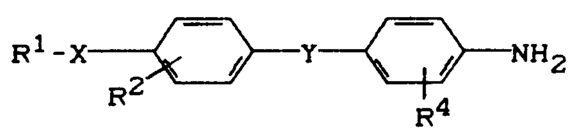

(VI)

in welcher

Y, X, $R^1$, $R^2$, $R^3$, $R^4$ die in (1) angegebene Bedeutung haben,

in an sich bekannter Weise diazotiert und anschließend mit Verbindungen der Formel (VII)

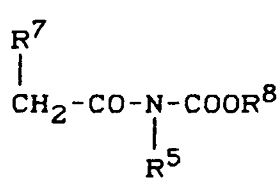

(VII)

in welcher

$R^5$, $R^7$, $R^8$ die in (5) angegebene Bedeutung haben,

umsetzt.

Die Verbindungen der Formel (I) sowie ihre Salze mit Basen oder Säuren sind hervorragend zur Bekämpfung parasitärer Protozoen und insbesondere von Coccidien sowie Fischparasiten geeignet.

Bevorzugte Verbindungen der Formel (I) sind Verbindungen, in denen

X für O oder S steht,

Y für O, S, CO,

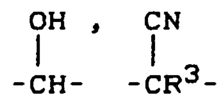

steht,

$R^1$ für $C_{1-4}$-Halogenalkyl steht,

$R^2$ für Wasserstoff, Halogen, $C_{1-4}$-Halogenalkyl steht,

$R^3$ für Wasserstoff, $C_{1-4}$-Alkyl steht,

$R^4$ für einen oder mehrere gleiche oder verschiedene Reste aus der Reihe Wasserstoff, Halogen, Halogenalkyl, $C_{1-4}$-Alkyl steht,

$R^5$ und $R^6$ für Wasserstoff stehen.

Besonders bevorzugt sind Verbindungen der Formel (I), in welcher

X für O oder S steht,

Y für O, S oder

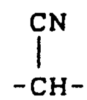

steht,

$R^1$ für $C_{1-4}$-Halogenalkyl steht,

$R^2$ für Wasserstoff, Halogen steht,

$R^4$ für einen oder mehrere gleiche oder verschiedene Reste aus der Reihe Wasserstoff, Halogen, $C_{1-4}$-Alkyl, Trifluormethyl steht,

$R^5$ und $R^6$ für Wasserstoff stehen.

Ganz besonders bevorzugt sind Verbindungen der Formel (I), in welcher

X für O oder S steht,

Y für O, S,

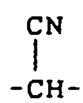

steht,

$R^1$ für $C_{1-4}$-Halogenalkyl, insbesondere Trifluormethyl, steht,

$R^2$ für Wasserstoff steht,

$R^4$ für einen oder mehrere gleiche oder verschiedene Reste aus der Reihe Wasserstoff, Fluor, Chlor, Brom, insbesondere Chlor, steht,

$R^5$ und $R^6$ für Wasserstoff steht.

Im einzelnen seien genannt

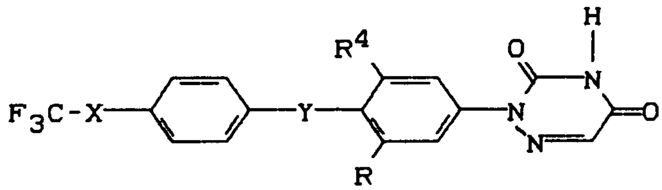

| X | Y | R | $R^4$ |
|---|---|---|---|
| O | O | Cl | Cl |
| S | O | Cl | Cl |
| O | S | Cl | Cl |
| S | S | Cl | Cl |
| O | CHCN | Cl | Cl |
| S | CHCN | Cl | Cl |

Weiterhin seien die folgenden Verbindungen genannt

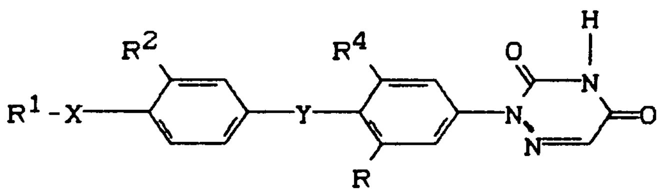

| X | Y | $R^1$ | $R^2$ | R | $R^4$ |
|---|---|---|---|---|---|
| O | O | $CHF_2$ | H | Cl | Cl |
| O | O | $CHF_2$ | Cl | Cl | Cl |
| O | O | $CF_2CHF_2$ | H | Cl | Cl |
| O | O | $CF_2CHF_2$ | Cl | Cl | Cl |
| O | O | $CF_3$ | H | $CH_3$ | $CH_3$ |
| O | S | $CHF_2$ | H | Cl | Cl |
| O | S | $CHF_2$ | Cl | Cl | Cl |
| O | S | $CF_2CHF_2$ | H | Cl | Cl |
| O | S | $CF_2CHF_2$ | Cl | Cl | Cl |
| O | S | $CF_3$ | Cl | $CH_3$ | $CH_3$ |
| O | CHCN | $CHF_2$ | H | Cl | Cl |
| O | CHCN | $CHF_2$ | Cl | Cl | Cl |
| O | CHCN | $CF_2CHF_2$ | H | Cl | Cl |
| O | CHCN | $CF_2CHF_2$ | Cl | Cl | Cl |
| O | CHCN | $CF_3$ | H | $CH_3$ | $CH_3$ |
| S | CHCN | $CF_3$ | H | $CH_3$ | $CH_3$ |
| S | O | $CHF_2$ | H | Cl | Cl |
| S | O | $CF_3$ | H | $CH_3$ | $CH_3$ |
| S | O | $CHF_2$ | Cl | Cl | Cl |
| S | O | $CF_2CHF_2$ | H | Cl | Cl |

Setzt man bei dem Verfahren 2a) zur Herstellung der Verbindungen der Formel (I), in welcher $R^5$ nicht für Wasserstoff steht, als Verbindung der Formel (Ia) 2-[4-(4'-Trifluormethylthiophenyl)-3,5-dichlorophenoxy]-1,2,4-triazin-3,5-(2,4, 4H)dion und als Verbindung der Formel (II) Methyliodid ein, läßt sich das Verfahren durch folgendes Schema beschreiben:

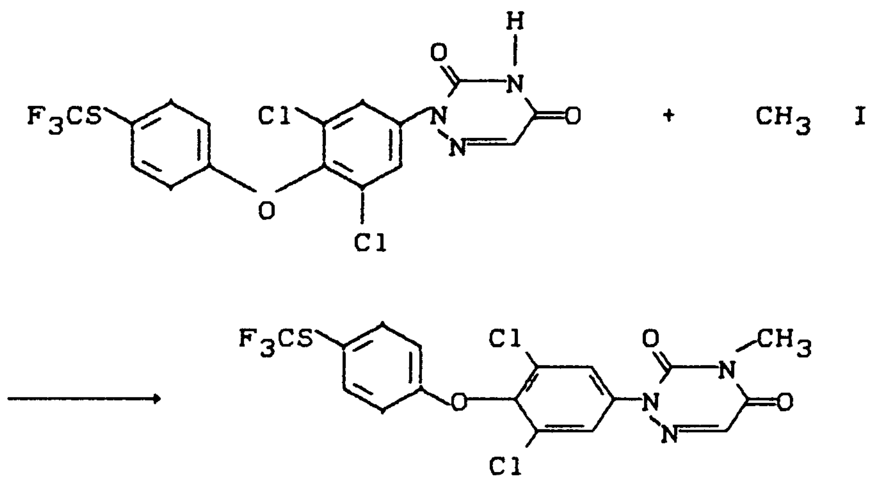


Die Verbindungen der Formel (Ia) werden wie bei Verfahren 2b) beschrieben dargestellt.

Die Verbindungen der Formel (II) sind bekannt oder lassen sich nach bekannten Methoden darstellen, Besonders genannt sei Methyliodid, Ethylbromid.

Das Verfahren wird durchgeführt, indem man eine Verbindung der Formel (Ia) in Gegenwart einer Base und eines Verdünnungsmittels mit Verbindungen der Formel (II) umsetzt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, Benzin, Ligroin, Benzol, Toluol, Xylol, Methylenchlorid, Ethylenchlorid, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutylketon, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Das Verfahren wird in Gegenwart von Basen durchgeführt. Als bevorzugte Basen seien genannt die Alkalihydroxide wie Natriumyhdroxid, Alkalialkoholate wie Natriummethylat oder Kaliumbutanolat, Metallhydride wie Natriumhydrid oder organische Basen wie 1,8-Diazabicyclo[5,40]-undec-7-en (DBU).

Das Verfahren wird durchgeführt bei Normaldruck und Temperaturen zwischen 20° und 140°C.

Die Reaktion wird durchgeführt indem man äquimolare Mengen der Verbindung der Formel (Ia) und Base zusammengibt, dieses Gemisch mit einer äquimolaren Menge der Verbindung der Formel (II) versetzt und auf die Reaktionstemperatur erhitzt.

Nach dem im folgenden beschriebenen Verfahren 2b) lassen sich sowohl die Verbindungen der Formel (I) als auch die Verbindungen der Formel (Ia) herstellen,

Wird bei Verfahren 2b) zur Herstellung der Verbindungen der Formel II als Verbindung der Formel VIII 2-[4-(4'-trifluormethylphenoxy)-1,2,4-triazin-2,5(2H,4H)dion-6-carbonsäure eingesetzt, läßt sich das Verfahren durch folgendes Schema beschreiben:

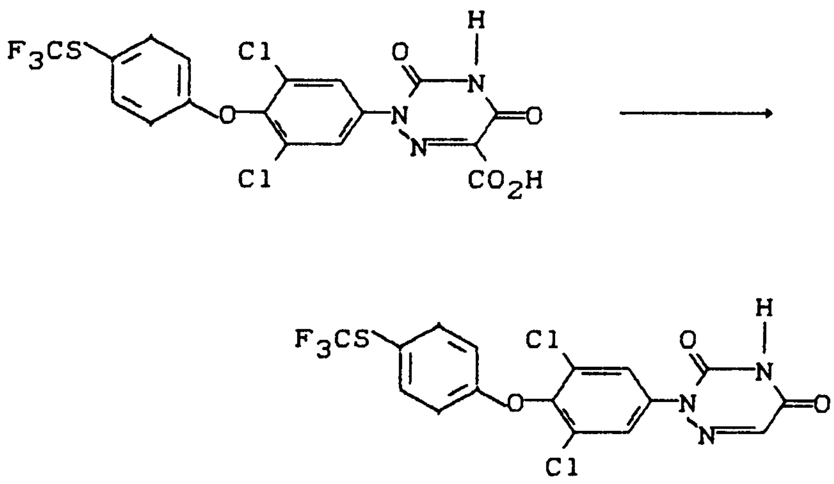

Die Verbindungen der Formel (III) werden nach dem weiter unten (4) beschriebenen Verfahren hergestellt. Es werden bevorzugt Verbindungen der Formel (III) eingesetzt, in denen Y, X, $R^1$, $R^2$, $R^3$, $R^4$, $R^5$ die bei den Verbindungen der Formel (I) angegebenen bevorzugten Bedeutungen haben,

Als Einzelverbindungen der Formel (III) seien genannt

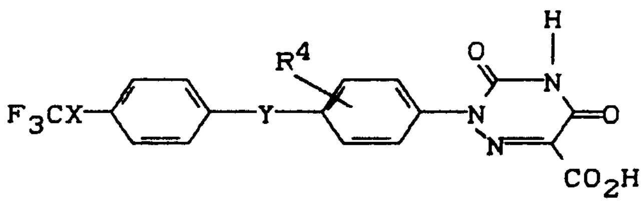

| X | Y | $R^4$ |
|---|---|---|
| O | O | 3,5-$Cl_2$ |
| S | O | 3,5-$Cl_2$ |
| O | S | 3,5-$Cl_2$ |
| S | S | 3,5-$Cl_2$ |
| O | CHCN | 3,5-$Cl_2$ |
| S | CHCN | 3,5-$Cl_2$ |

Die Decarboxylierung wird gegebenenfalls in Gegenwart von inerten organischen Verdünnungsmitteln durchgeführt. Hierzu gehören aliphatische und aromatische, gegebenenfalls halogenierte Kohlenwasserstoffe wie Nonan, Decan, Dodecan, Xylole, Alkohole wie Diethylenglykol, Ether wie Ethylenglykolmonobutylether, Diethylenglykoldibutylether, Sulfoxide wie Dimethylsulfoxid und Sulfone wie Tetramethylensulfon.

Darüber hinaus kann die Reaktion in Gegenwart von mercaptogruppenhaltigen Carbonsäuren wie z.B. Mercaptoessigsäure oder Thiosalicylsäure durchgeführt werden.

Die Umsetzung erfolgt bei Temperaturen zwischen 150 und 300 ° C, gegebenenfalls in Gegenwart von mercaptogruppenhaltigen Carbonsäuren wie z.B. Mercaptoessigsäure vorzugsweise zwischen 160 und 250 ° C, insbesondere zwischen 180 und 210 ° C.

Es wird bei Normaldruck gearbeitet. Die Verbindungen der Formeln (III) werden in Substanz oder im jeweiligen Verdünnungsmittel, gelöst oder suspendiert, erhitzt.

Setzt man bei dem Verfahren 2c als Verbindung der Formel Ib 2-[4-(4'-Trifluormethylthiophenyl)-3,5-dichlorophenoxy]-1,2,4-triazin-3,5-[2,4,4H)dion ein, läßt sich das Verfahren durch folgendes Formelschema beschreiben:

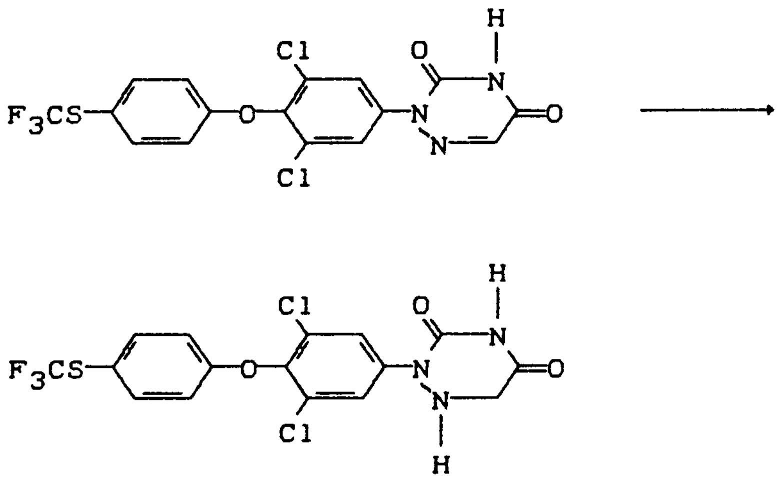

Die Verbindungen der Formel Ib sind neu und werden z.B. nach den Verfahren 2a-c) erhalten.

Bevorzugt seien Verbindungen der Formel Ib genannt, in denen X, Y, $R^1$, $R^2$, $R^3$, $R^4$, $R^5$ die bei den Verbindungen der Formel I genannten bevorzugten Bedeutungen haben.

Im einzelnen seien folgende Verbindungen der Formel Ib genannt:

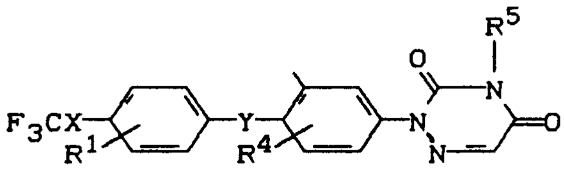

| X | Y | $R^1$ | $R^4$ |
|---|---|---|---|
| O | O | Cl | Cl |
| S | O | Cl | Cl |
| O | S | Cl | Cl |
| S | S | Cl | Cl |
| O | CHCN | Cl | Cl |
| S | CHCN | Cl | Cl |
| $SO_2$ | CHCN | Cl | Cl |

Das Verfahren 2c) wird durchgeführt, indem man eine Verbindung der Formel Ib in Gegenwart eines Reduktionsmittels und einer Säure erwärmt. Als Reduktionsmittel können Metalle wie z.B. Zink und Metallsalze wie z.B. Zinn(II)chlorid, Metallhydride wie Lithiumaluminiumhydrid und katalytisch angeregter Wasserstoff verwendet werden.

Als Säuren kommen verdünnte Mineralsäuren wie z.B. Salzsäure und organische Säuren wie z.B. Eisessig zum Einsatz. Die Reaktion kann gegebenenfalls in Gegenwart eines Verdünnungsmittels durchgeführt werden. Als Verdünnungsmittel können inerte organische Lösungsmittel verwendet werden. Hierzu gehören Kohlenwasserstoffe wie z.B. Toluol, Ether wie z.B. Dioxan, Ketone wie z.B. Aceton und Alkohole wie z.B. Ethanol. Die Reduktion erfolgt bei Temperaturen zwischen 80 und 120 °C bei Normaldruck oder erhöhtem Druck.

Verfahren 2d und 2e werden nach den bei Verfahren 2a angegebenen Bedingungen durchgeführt.

Wird im Verfahren 4 zur Herstellung der Verbindungen der Formel (III) als Verbindung der Formel (IV) 2-[4-(4'-trifluormethylthiophenyl-3,5-dichloro-phenoxy)]-6-cyano-1,2,4-triazin-3,5(2H,4H)dion eingesetzt, läßt sich das Verfahren durch folgendes Formelschema beschreiben:

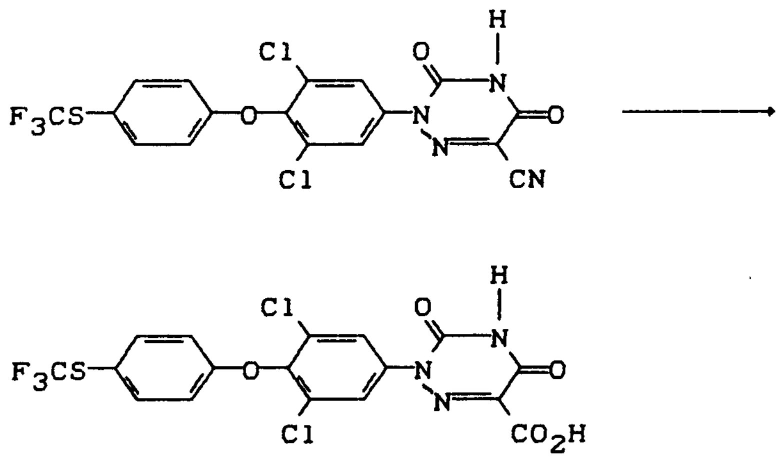

Die Verbindungen der Formel (IV) sind neu. Ihre Herstellung erfolgt nach dem unter (6) beschriebenen Verfahren.

Bevorzugt werden Verbindungen der Formel (IV) eingesetzt, in der Y, X, $R^1$, $R^2$, $R^3$, $R^4$, $R^5$ die bei den Verbindungen der Formel (I) angegebenen bevorzugten Bedeutungen hat und $R^7$ für CN steht.

Die Hydrolyse der Verbindungen der Formel (VI) wird unter sauren Bedingungen durchgeführt. Als Säuren kommen Mineralsäuren zum Einsatz wie z.B. Salzsäure, Bromwasserstoffsäure, Schwefelsäure und Gemische aus Mineralsäuren und organischen Säuren wie z.B. Essigsäure oder Propionsäure.

Die Umsetzung erfolgt bei Temperaturen zwischen 80 und 120 ° C. Es wird unter Normaldruck gearbeitet.

Die Verbindungen der Formel (IV) werden im 10-30-fachen Volumen der Säure oder des Säuregemisches gelöst und bis zur abgeschlossenen Hydrolyse erhitzt.

Wird im Verfahren 6 zur Herstellung der Verbindungen der Formel (IV) als Verbindung der Formel XI Ethyl-N-[[[Cyano(3,5-dichlor-(4'-trifluormethylphenyl)phenoxy-hydrazinyliden]methyl]carbonyl]-carbamat eingesetzt, läßt sich das Verfahren durch folgendes Schema beschreiben:

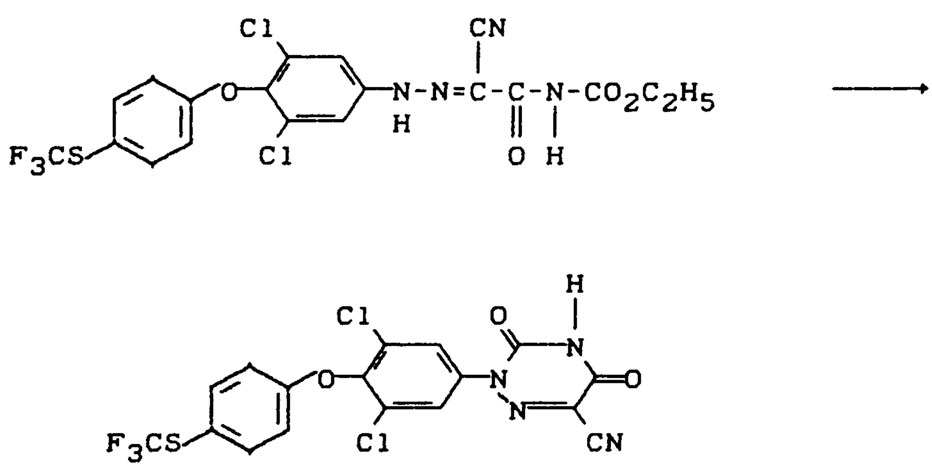

Die Verbindungen der Formel (V) sind neu. Ihre Herstellung erfolgt nach dem unter (8) beschriebenen Verfahren. Bevorzugt werden Verbindungen der Formel (V) eingesetzt, in der Y, X, $R^1$, $R^2$, $R^3$, $R^4$, $R^5$ die bei den Verbindungen der Formel (I) angegebenen bevorzugten Bedeutungen haben, $R^8$ für $C_{1-4}$-Alkyl, insbesondere Methyl oder Ethyl und Phenyl steht, und $R^7$ für CN steht.

Als Einzelverbindungen der Formel (V) seien genannt

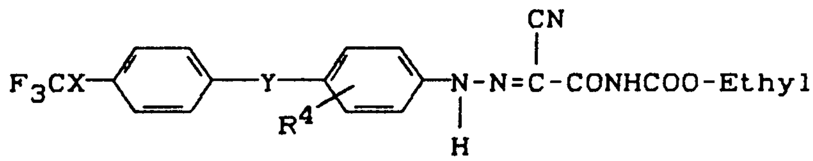

| X | Y | $R^4$ |
|---|---|---|
| O | O | 3,5-$Cl_2$ |
| S | O | 3,5-$Cl_2$ |
| O | S | 3,5-$Cl_2$ |
| S | S | 3,5-$Cl_2$ |
| O | CHCN | 3,5-$Cl_2$ |
| S | CHCN | 3,5-$Cl_2$ |

Das Verfahren 6) wird durchgeführt, indem man eine Verbindung der Formel (V) erhitzt, gegebenenfalls in Gegenwart eines Lösungsmittels und einer Base`

Als Lösungsmittel und Basen finden die bei der Herstellung der Verbindungen I aufgeführten Lösungsmittel und Basen Verwendung. Als weitere besonders bevorzugte organische Lösungsmittel werden Alkohole wie z.B. Ethanol oder organische Säuren wie z.B. Eisessig eingesetzt.

Besonders bevorzugte Basen sind die Hydroxide und Acetate der Alkali- oder Erdalkalimetalle wie z.B. NaOH oder Natrium- und Kaliumacetat.

Die Umsetzung erfolgt unter Normaldruck bei Temperaturen zwischen 70 und 150 °C, vorzugsweise zwischen 70 und 100 °C.

Die verwendete Base wird in 10-80 %igem molarem Überschuß eingesetzt. Das Reaktionsgemisch wird nach abgeschlossener Cyclisierung vorzugsweise mit einer verdünnten Mineralsäure wie z.B. Salzsäure angesäuert und das als Feststoff anfallende Produkt abfiltriert.

Die bei Verfahren 6) eingesetzten Verbindungen der Formel VIII sind neu. Sie werden nach dem unter (8) beschriebenen Verfahren hergestellt.

Wird im Verfahren 8) zur Herstellung der Verbindungen der Formel (V) als Verbindung der Formel (VI) 4-(4'-Trifluormethylthiophenyl-3,5-dichloroanilin eingesetzt und als Verbindung der Formel (VII) Ethyl-cyanacetylurethan läßt sich das Verfahren durch folgendes Schema beschreiben:

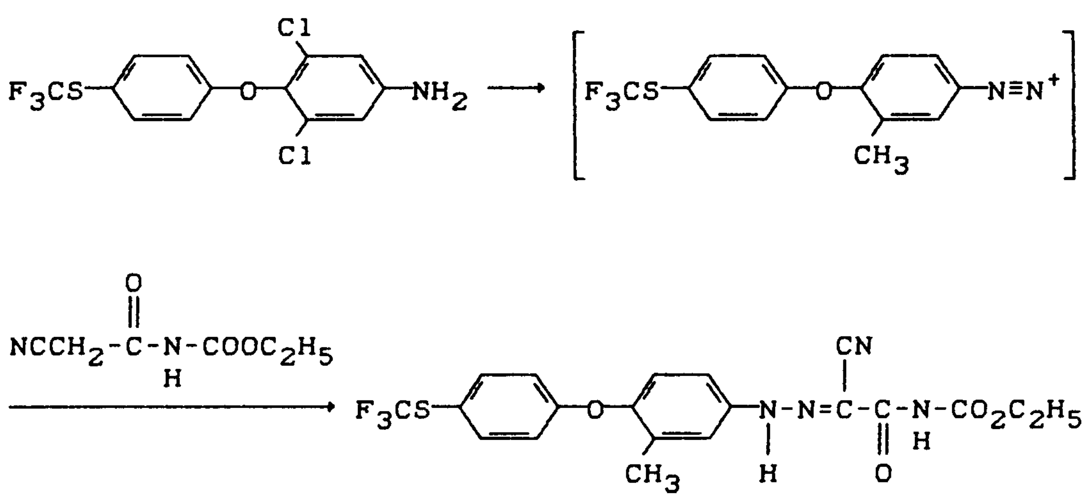

Die Verbindungen der Formeln (VI) und (VII) sind bekannt oder lassen sich analog zu bekannten Verfahren herstellen (vgl. DE-OS 24 13 722; 27 18 799; US-P 4 005 218; Le A 26 382).

Das Verfahren wird durchgeführt, indem man ein Anilin der Formel (VI) mit $NaNO_2$ und konz. Mineralsäure wie z.B. HCl, gegebenenfalls in Gegenwart eines Verdünnungsmittels, umsetzt.

Als Verdünnungsmittel dienen mit Wasser mischbare Verdünnungsmittel wie Alkohole, z.B. Methanol,

Ethanol, Glycolether wie Monomethylglycolether, Nitrile wie Acetonitril, Dimethylsulfoxid, organische Säuren wie z.B. Eisessig, Ameisensäure, Propionsäure oder Gemische organischer Säuren, vorzugsweise ein Gemisch aus Eisessig und Propionsäure.

Das so erzeugte Diazoniumsalz wird in situ mit einer Verbindung der Formel (VII) wie z.B. Malonyldiurethan oder Cyanacetylurethan in Gegenwart einer Base umgesetzt. Als Basen finden Verwendung Hydroxide und Carbonate der Alkali und Erdalkalimetalle sowie Acetate von Natrium, Kalium und Ammonium.

Weiterhin können organische Basen wie Pyridin oder Triethylamin verwendet werden.

Die Diazotierung wird bei Normaldruck und bei Temperaturen zwischen 0 ° C und 10 ° C durchgeführt. Die Zugabe der Verbindungen der Formel (VII) erfolgt bei 0 ° bis 20 ° C. Anilin und Nitrit werden in äquimolaren Mengen in einem Überschuß Säure vorzugsweise der 2-3 fachen molaren Menge umgesetzt. Die CH-acide Verbindung wird in 0 bis 30 %igem molaren Überschuß, vorzugsweise 10 %igem Überschuß zugesetzt. Die Base wird im 1,5-2,5-fachen molaren Überschuß zugesetzt. In vorteilhafter Weise kann auch eine reverse Addition durchgeführt werden, d. h. das durch Diazotierung erzeugte Diazoniumsalz der Verbindung der Formel (VI) wird bei 0 ° bis 10 ° C zu einer Mischung einer Verbindung der Formel (VII) und dem jeweiligen Lösungsmittel bzw. -Gemisch zugetropft.

Das Kupplungsprodukt aus Diazoniumsalz und CH-acider Verbindung ist im Reaktionsmedium unlöslich und kann als Feststoff isoliert werden.

Das Verfahren kann auch so geführt werden, daß ohne Isolierung der Verbindung der Formel (V) direkt Verbindungen der Formel (VI) entstehen. Dazu wird die Diazotierung der Aniline der Formel (VI) und die Umsetzung mit den Urethanen der Formel (VII) in einem für die Cyclisierung geeigneten Verdünnungsmittel durchgeführt. Das Reaktionsgemisch wird nach erfolgter Diazotierung und Kupplung erwärmt und dann das Triazindion der Formel (IV) isoliert.

Als Verdünnungsmittel seien genannt: Alkohole wie Methanol, Ethanol.

Zur Cyclisierung wird das Reaktionsgemisch auf ca, 80 bis 120 ° C, bevorzugt ca, 80 bis 100 ° C, erwärmt.

Die Aufarbeitung erfolgt wie weiter oben bei Verfahren (6) zur Herstellung der Verbindungen der Formel (IV) angegeben.

Die Wirkstoffe eignen sich bei günstiger Warmblütertoxizität zur Bekämpfung von parasitischen Protozoen die in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Stämme wirksam. Durch die Bekämpfung der parasitischen Protozoen sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist.

Zu den parasitischen Protozoen zählen:
Mastigophora (Flagellata) wie z.B. Trypanosomatidae z.B. Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie z.B. Trichomonadidae z.B. Giardia lamblia, G. canis.

Sarcomastigophora (Rhizopoda) wie Entamoebidae z.B. Entamoeba histolytica, Hartmanellidae z.B. Acanthamoeba sp., Hartmanella sp.

Apicomplexa (Sporozoa) wie Eimeridae z.B. Eimeria acervulina, E. adenoides, E. alabahmensis, E. anatis, E. anseris, E. arloingi, E. ashata, E. auburnensis, E. bovis, E. brunetti, E. canis, E. chinchillae, E. clupearum, E. columbae, E. contorta, E. crandalis, E. dabliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I.rivolta, I. spec., I. suis, Cystisospora spec., Cryptosporidium spec. wie Toxoplasmadidae z.B. Toxoplasma gondii, wie Sarcocystidae z.B. Sarcocystis bovicanis, S. bovihominis, S. ovicanis, S. ovifelis, S. spec., S. suihominis wie Leucozoidae z.B. Leucozytozoon simondi, wie Plasmodiidae z.B. Plasmodium berghei, P. falciparum, P. malariae, P. ovale, P vivax, P. spec., wie Piroplasmea z.B. Babesia argentina, B. bovis, B. canis, B. spec., Theileria parva, Theileria spec., wie Adeleina z.B. Hepatozoon canis, H. spec.

Ferner Myxospora und Microspora z.B. Glugea spec. Nosema spec.

Ferner Pneumocystis carinii, sowie Ciliophora (Ciliata) wie z.B. Balantidium coli, Ichthiophthirius spec., Trichodina spec., Epistylis spec.

Die erfindungsgemäßen Verbindungen sind auch wirksam gegen Protozoen, die als Parasiten bei Insekten auftreten. Als solche seien genannt Parasiten des Stammes Microsporida, insbesondere der

Gattung Nosema. Besonders genannt sei Nosema apis bei der Honigbiene.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Vogelarten für Heim- und Zoohaltung. Ferner gehören dazu Nutz- und Zierfische.

Zu Labor- und Versuchstieren gehören Mäuse, Ratten, Meerschweinchen, Goldhamster, Hunde und Katzen.

Zu den Hobbytieren gehören Hunde und Katzen.

Zu den Fischen gehören Nutz-, Zucht-, Aquarien- und Zierfische aller Altersstufen, die in Süß- und Salzwasser leben. Zu den Nutz- und Zuchtfischen zählen z. B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabream (Pagurus major), Seabass (Dicentrarchus labrax), Grey mullet (Mugilus cephalus), Pompano, Gilthread seabream (Sparus auratus), Tilapia spp., Chichliden-Arten wie z. B. Plagioscion, Channel catfish. Besonders geeignet sind die erfindungsgemäßen Mittel zur Behandlung von Fischbrut, z. B. Karpfen von 2 - 4 cm Körperlänge. Sehr gut geeignet sind die Mittel auch in der Aalmast.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; Halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden

sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgießformulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8/C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $c_{16}$-$c_{18}$,Isopropylmyristat, Isopropylpalmitat, Capryl/Caprinsäureester von gesättigten Fettalkoholen der Kettenlänge $c_{12}$-$c_{18}$, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:

Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;

ampholytische Tenside wie Di-Na-N-lauryl-β-iminodipropionat oder Lecithin;

anionaktive Tenside, wie Na-Laurylsulfat, Fettalkoholethersulfate, Mono/Dialkylpolyglykoletherorthophosphorsäureester-monoethanolaminsalz;

kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel,

Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen vorliegen.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 1 bis 50 Gewichtsprozent.

Im allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

Die Wirkstoffe können auch zusammen mit dem Futter oder Trinkwasser der Tiere verabreicht werden.

Futter- und Nahrungsmittel enthalten 0,01 bis 100 ppm, vorzugsweise 0,5 bis 50 ppm des Wirkstoffs in Kombination mit einem geeigneten eßbaren Material.

Ein solches Futter- und Nahrungsmittel kann sowohl für Heilzwecke als auch für prophylaktische Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem eßbaren organischen oder anorganischen Täger enthält mit üblichen Futtermitteln. Eßbare Träger sind z.B. Maismehl oder Mais- und Sojabohnenmehl oder Mineralsalze, die vorzugsweise eine geringe Menge eines eßbaren Staubverhütungsöls, z.B. Maisöl oder Sojaöl, enthalten. Die hierbei erhaltene Vormischung kann dann dem vollständigen Futtermittel vor seiner Verfütterung an die Tiere zugesetzt werden.

Beispielhaft sei der Einsatz bei der Coccidiose genannt:

Für die Heilung und Prophylaxe etwa der Coccidiose bei Geflügel, insbesondere bei Hühnern, Enten, Gänsen und Truthähnen, werden 0,1 bis 100 ppm, vorzugsweise 0,5 bis 100 ppm eines Wirkstoffs mit einem geeigneten eßbaren Material, z.B. einem nahrhaften Futtermittel, gemischt. Falls gewünscht, können diese Mengen erhöht werden, besonders wenn der Wirkstoff vom Empfänger gut vertragen wird. Entsprechend kann die Verabreichung über das Trinkwasser erfolgen.

Für die Behandlung von Einzeltieren, z.B. im Falle der Behandlung der Coccidiose bei Säugetieren oder der Toxoplasmose, werden vorzugsweise Wirkstoffmengen von 0,5 bis 100 mg/kg Körpergewicht täglich verabreicht, um die gewünschten Ergebnisse zu erzielen. Trotzdem kann es zeitweilig notwendig sein, von den genannten Mengen abzuweichen, insbesodnere in Abhängigkeit vom Körpergewicht des Versuchstieres oder der Art der Verabreichungsmethode, aber auch wegen der Tiergattung und seiner individuellen Reaktion auf den Wirkstoff oder der Art der Formulierung und der Zeit oder dem Abstand, zu dem er verabreicht wird. So kann es in gewissen Fällen genügen, mit weniger als der vorstehend genannten Mindestmenge auszukommen, während in anderen Fällen die genannte obere Grenze überschritten werden muß. Bei der Verabreichung größerer Mengen kann es zweckmäßig sein, diese im Verlauf des Tages in mehrere Einzeldarreichungen zu unterteilen.

Darüber hinaus sind die erfindungsgemäßen Verbindungen wirksam gegenüber verschiedenen zu den Helminthen (Würmern) zählenden Fischparasiten.

Zu den Parasiten bei Fischen gehören aus dem Unterreich der Protozoen Spezies des Stammes der Ciliata, z.B. Ichthyophthirius multifiliis, Chilodonella cyprini, Trichodina spp., Glossatella spp., Epistylis spp. des Stammes der Myxosporidia, z.B. Myxosoma cerebralis, Myxidium spp., Myxobolus spp., Heneguya spp., Hoferellus spp., der Klasse der Mikrosporidia z.B. Glugea spp., Thelohania spp., Pleistophora spp., aus dem Stamm der Plathelminthen: Trematoden; Monogenea z.B. Dactylogyrus spp., Gyrodactylus spp., Pseudodactylogyrus spp., Diplozoon spp., Cestoden, z.B. aus den Gruppen der Caryphyllidea (z.B. Caryophyllaeus laticeps), Pseudophyllidea (z.B. Diphyllobothrium spp.), Tetraphyllidea (z.B. Phyllobothrium spp.) und Protocephalida (z.B. Arten der Gattung Proteocephalus) und aus dem Stamm der Arthropoda verschiedene parasitische Crustaceen, insbesondere aus den Unterklassen der Branchiura (Fischläuse) und Copepoda (Ruderfußkrebse) sowie den Ordnungen der Isopoda (Asseln) und Amphipoda (Flohkrebse).

Die Behandlung der Fische erfolgt entweder oral, z. B. über das Futter oder durch Kurzzeitbehandlung, "medizinisches Bad", in das die Fische eingesetzt und in dem sie eine Zeitlang (Minuten bis mehrere Stunden) z. B. beim Umsetzen von einem Zuchtbecken zum anderen gehalten werden.

Es kann aber auch eine vorübergehende oder dauernde Behandlung des Lebensraums der Fische (z. B. ganzer Teichanlagen, Aquarien, Tanks oder Becken), in denen die Fische gehalten werden, erfolgen.

Der Wirkstoff wird in Zubereitungen verabreicht, die den Anwendungen angepaßt sind.

Die Konzentration des Wirkstoffs, liegt in den Zubereitungen bei 1 ppm bis 10 Gew.-%.

Bevorzugte Zubereitungen zur Kurzzeitbehandlung in der Anwendung als "medizinisches Bad" z.B. bei der Behandlung beim Umsetzen der Fische oder zur Behandlung des Lebensraums (Teichbehandlung) der Fische sind Lösungen des Wirkstoffs in einem oder mehreren polaren Lösungsmitteln, die bei Verdünnen mit Wasser alkalisch reagieren.

Zur Herstellung dieser Lösungen wird der Wirkstoff in einem polaren, wasserlöslichen Lösungsmittel gelöst, welches entweder alkalisch reagiert oder dem eine alkalische wasserlösliche Substanz zugefügt wird. Letztere wird vorteilhaft ebenfalls im Lösungsmittel gelöst, kann aber auch in dem Lösungsmittel suspendiert sein und sich erst im Wasser lösen. Dabei soll das Wasser nach Zusatz der Wirkstofflösung einen pH-Wert von 7-10, vorzugsweise aber einen pH-Wert von 8-10 haben.

Die Konzentration des Wirkstoffes kann im Bereich von 0,5-50 % liegen, vorzugsweise aber in einem Bereich von 1-25 %.

Als Lösungsmittel kommen alle wasserlöslichen Lösungsmittel in Betracht, in denen der Wirkstoff in genügender Konzentration löslich ist und die physiologisch unbedenklich sind.

Dies sind Ethylalkohol, Isopropylalkohol, Benzylalkohol, Glycerin, Propylenglykol, Polyethylenglykole, Poly(oxoethylen)-poly(oxypropylen)-Polymere, basische Alkohole wie Mono-, Di- und Triethanolamin, Ketone wie Aceton oder Methylethylketon, Ester wie Milchsäureethylester ferner N-Methylpyrrolidon, Dimethylacetamid, Dimethylformamid, ferner Dispergier- und Emulgiermittel wie polyoxyethyliertes Rizinusöl, Polyethylenglykol-Sorbitan-Monooleat, Polyethylenglykolstearat, oder Polyethylenglykolether, Polyethylenglykol-Alkylamine.

Als Basen zur Einstellung des alkalischen pH-Wertes seien genannt organische Basen wie basische Aminosäuren wie L- bzw. D,L-Arginin, L- bzw. D, L-Lysin, Methylglucosamin, Glucosamin, 2-Amino-2-hydroxymethylpropan-diol-(1,3) ferner wie N,N,N',N'-tetrakis-(2-hydroxypropyl)-ethylendiamin oder Polyether-Tetrol auf der Basis Ethylendiamin (M.G. 480-420), anorganische Basen, wie Ammoniak oder Natriumcarbonat-gegebenenfalls unter Zugabe von Wasser.

Die Zubereitungen können auch 0,1 bis 20 Gew.-%, vorzugsweise 0,1-10 Gew.-% anderer Formulierhilfsstoffe, wie Antioxydantien, Tenside, Suspensionsstabilisatoren und Verdickungsmittel wie z.B. Methylcellulose, Alginate, Polysaccharide, Galaktomannane und kolloidale Kieselsäure enthalten. Der Zusatz von Farbe, Aroma und Aufbaustoffen zur Tierernährung ist ebenfalls möglich. Auch Säuren, die mit der vorgelegten Base zusammen ein Puffersystem bilden oder den pH der Lösung reduzieren, sind hier zu nennen.

Die Konzentration des Wirkstoffs bei der Anwendung hängt ab von Art und Dauer der Behandlung, sowie Alter und Zustand der behandelten Fische. Sie beträgt z.B. bei Kurzzeitbehandlung 2-50 mg Wirkstoff pro Liter Wasser bevorzugt 5-10 mg pro Liter, bei einer Behandlungsdauer von 3-4 Stunden` Bei der Behandlung von jungen Karpfen wird z.B. mit einer Konzentration von 5-10 mg/l und einer Behandlungsdauer von ca. 1-4 Stunden gearbeitet.

Aale werden mit Konzentrationen von ca, 5 mg/l ca. 4 Stunden behandelt.

Bei längerer Behandlungsdauer oder bei Dauerbehandlung kann die Konzentration entsprechend niedriger gewählt werden.

Bei Teichbehandlungen können 0,1-5 mg Wirkstoff pro Liter Wasser verwendet werden.

Zubereitungen zur Anwendung als Futterzusatz sind z.B. wie folgt zusammengesetzt:

a)

| | | | |
|---|---|---|---|
| Wirkstoff der Formel I | 1 | - 10 | Gewichtsteile |
| Sojabohnen-Protein | 49 | - 90 | Gewichtsteile |

b)

| | | | |
|---|---|---|---|
| Wirkstoff der Formel I | 0,5 | - 10 | Gewichtsteile |
| Benzylalkohol | 0,08 | - 1,4 | Gewichtsteile |
| Hydroxypropyl-methylcellulose | 0 | - 3,5 | Gewichtsteile |
| Wasser | | Rest ad 100 | |

Zubereitungen zur Anwendung bei "medizinischen Bädern" und zur Teichbehandlung sind z.B. wie folgt zusammengesetzt und hergestellt.

c) 2,5 g Wirkstoff der Formel (I) werden in 100 ml Triethanolamin unter Erwärmen gelöst.

d) 2,5 g Wirkstoff der Formel (I)

12,5 g Milchsäure werden in 100 ml Triethanol amin unter Erwärmen und Rühren gelöst.

e) 10,0 g Wirkstoff der Formel (I) wird in 100 ml Monoethanolamin gelöst.

(f)

| | |
|---|---|
| Wirkstoff der Formel I | 5,0 g |
| Propylenglykol | 50,0 g |
| Natriumcarbonat | 5,0 g |
| Wasser ad | 100 ml |

(g)

| | |
|---|---|
| Wirkstoff der Formel I | 5,0 g |
| Monoethanolamin | 10 g |
| N-Methylpyrrolidon ad | 100 ml |

(h)

| | |
|---|---|
| Wirkstoff der Formel I | 2,5 g |
| Natriumcarbonat | 5,0 g |
| Polyethylenglykol200 ad | 100 ml |

Der Wirkstoff wird unter Erwärmen im Polyethylenglykol gelöst und Natriumcarbonat darin suspendiert.

Beispiel A

Coccidiose bei Hühnern

9 bis 11 Tage alte Küken wurden mit 40000 sporulierten Oozysten von stark virulenten Stämmen von Eiveria acervulina, E. maxima und E. tenella, den Krankheiteserregern der intestinalen Coccidiose infiziert`

Von 3 Tagen vor der Infektion bis 8 Tage nach der Infektion (Ende des Versuchs) wurde Wirkstoff in der angegebenen Konzentration im Futter der Tiere eingemischt verabreicht.

Die Zahl der Oozysten im Kot wurde mit Hilfe der McMaster-Kammer bestimmt (siehe Engelbrecht und Mitarbeiter "Parasitologische Arbeitsmehoden in Medizin und Veterinärmedizin", S. 172, Akademie-Verlag, Berlin (1965)).

Als wirksam werden diejenigen Dosen angesehen, die die Ausscheidung von Oozysten und/oder klinische Symptome der Coccidiose einschließlich der Mortalität vollständig oder in hohem Maße verhüteten. In der folgenden Tabelle werden die wirksamen Dosen angegeben:

<u>Tabelle 1</u>

Coccidiose bei Hühnern

| Beispiel Nr. | Dosis ppm. | Sterberate tot/eingesetzt | Oocystenausscheidung in % im Vergleich zur unbehandelten infizierten Kontrolle | Gewichtszunahme in % im Vergleich zur nicht infizierten unbehandelten Kontrolle | Blutausscheidung mit dem Kot |
|---|---|---|---|---|---|
| unbehandelte infizierte Kontrolle | | 2/6 | 100 | 35 | stark |
| 3 | 50 | 0/3 | 0 | 100 | keine |
| 4b | 1 | 0/3 | 0 | 100 | " |

Herstellbeispiele

I Beispiel für Verfahren 2a)

Beispiel 1

2-[4-(4-Trifluormethylphenyl)phenoxy]-3-N-methyl-3-N-methyl-3,5-(2H,4H)-dioxo-1,2,4-triazin

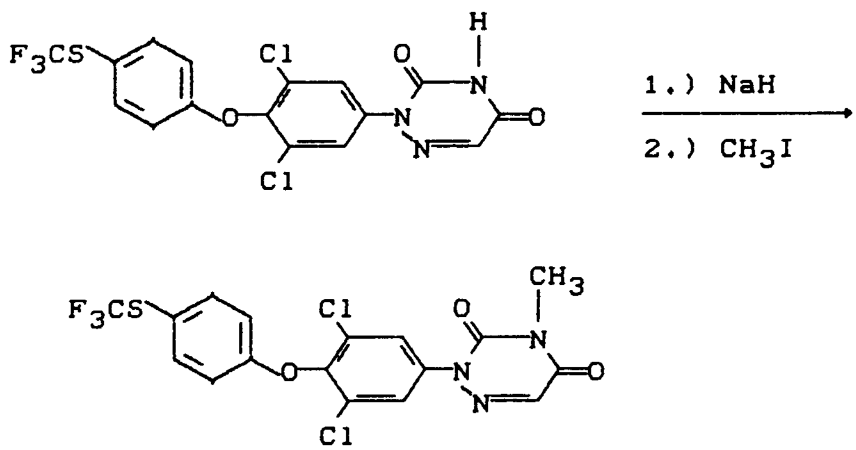


3,1 g (7 mmol) Azauracil werden in 20 ml absolutem DMSO gelöst und mit 0,16 g (6 mmol Natriumhydrid versetzt. Man rührt 20 min bei RT und gibt dann 1,5 g (9 mmol) Methyljodid in 5 ml DMSO unter Argon zu, Man erwärmt auf 50 ° C und hält 3 h bei dieser Temperatur. Anschließend wird das Reduktionsgemisch i, V, eingeengt und dann mit Wasser versetzt. Nach dem Absaugen des ausgefallenen Feststoffs erhält man so 2,3 g (72 % der Theorie) der N-Methylverbindung.

II Beispiel für Verfahren 2b)

Beispiel 2

2-(4-(4'-Trifluormethylphenyl)-3,5-dichlorophenoxy]-1,2,4-triazin-3,5(2,4)dion

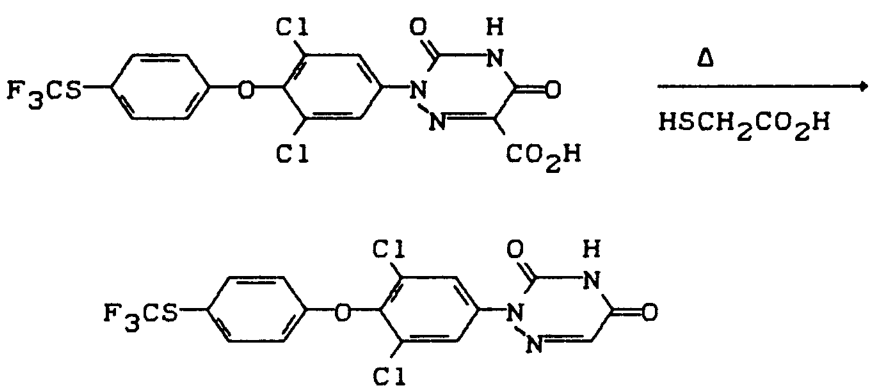


14,8 g (0,03 mol) Carbonsäure werden in 20 ml Mercaptoessigsäure auf 170 ° C erhitzt. Nach 1,5 h läßt man abkühlen, versetzt mit Wasser und erhält nach Abfiltration 11,5 g (85 % der Theorie) decarboxyliertes Produkt.

Analog werden hergestellt

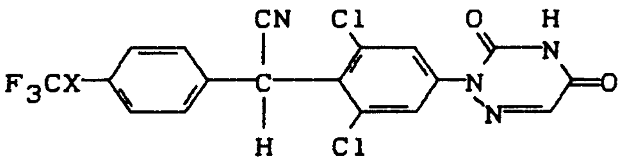

| | | |
|---|---|---|
| Bsp. 3<br>Bsp. 4 | X = O<br>X = S | Fp. 110 °C<br>Fp. 191 °C |

II Beispiel für Verfahren 2c)

Beispiel 4a

10 g (23 mmol) 2-[4'-Trifluormethylthiophenyl)-3,5-dichloro-phenoxy]-1,2,4-triazin-3,5(2H, 4H)dion werden mit 10 g Zink in 100 ml Eisessig 1,5 h unter Rückfluß gerührt.

Anschließend wird heiß abgesaugt und der Rückstand zweimal mit DMF ausgekocht. Die Filtrate werden eingeengt mit Wasser verrührt und der ausgefallene Feststoff abgesaugt. Man erhält so 7,8 g (78 % d.Th.) der Dihydroverbindung als farblosen Feststoff.

Analog werden hergestellt

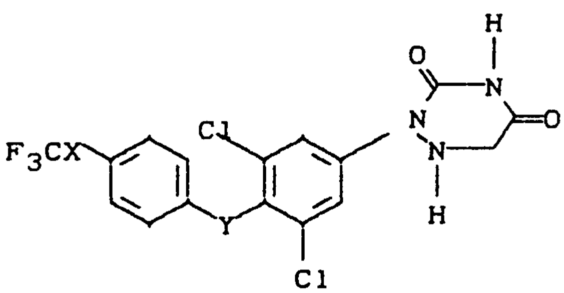

| Bsp. Nr. | X | Y | Fp |
|---|---|---|---|
| 4b | O | CHCN | |
| 4c | S | CHCN | |
| 4d | S | O | |

III Beispiel für Verfahren 4

Beispiel 5

2-[4-(4'-Trifluormethylthiophenyl)phenoxyl-3,5-(2H,4H)-dioxo-1,2,4-triazin-6-carbonsäure

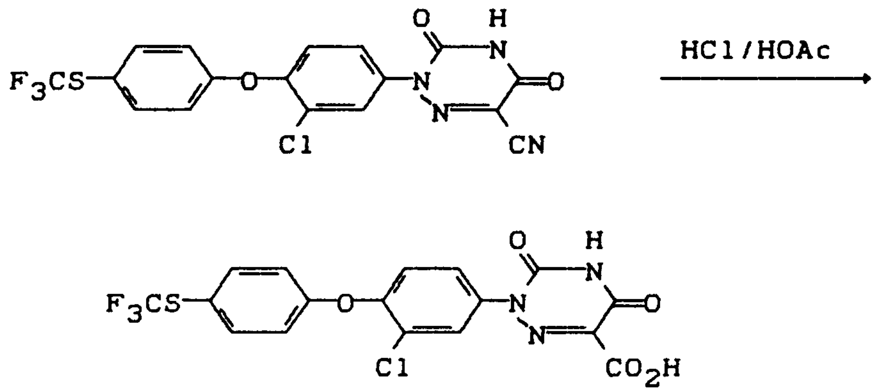


8,5 g (0,02 mol) Cyanazauracil werden in 50 ml Eisessig und 50 ml HCl konz. 4 Stunden gekocht. Anschließend kühlt man ab und verdünnt mit Wasser. Der ausgefallene Feststoff wird abgesaugt und getrocknet. Man erhält so 6,6 g (74 %) der Carbonsäure.

Analog werden hergestellt

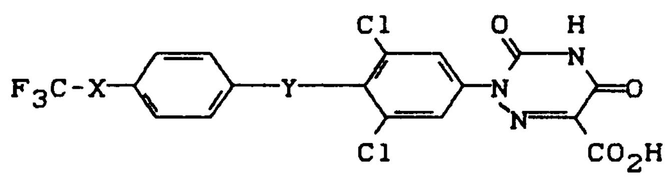

| | | |
|---|---|---|
| Bsp. 6 | X = O | Y = CHCN |
| Bsp. 7 | X = S | Y = CHCN |

IV Beispiel für Verfahren 6

<u>Beispiel 8</u>

2-[4-(4'-Trifluormethylthiophenyl)phenoxyl-3,5-(2H,4H),dioxo-6-cyano-1,2,4-triazin

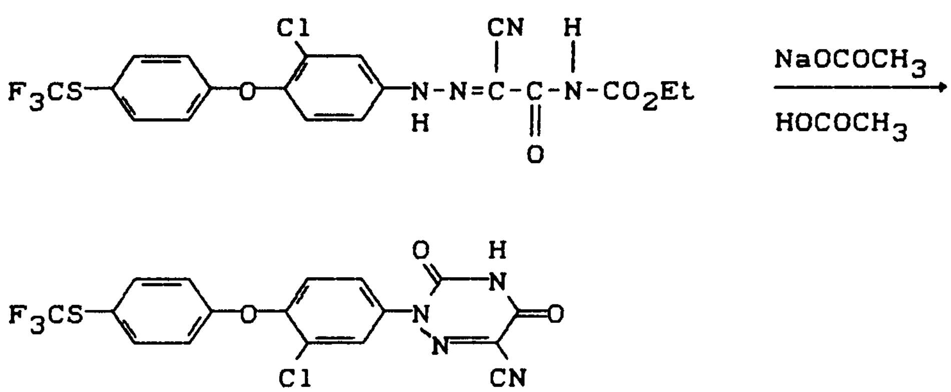

15 g (0.029 mol) des Hydrazonocyanurethans, 3,3 g (0,44 mol) Natriumacetat werden in 50 ml Eisessig 2 h unter Rückfluß erhitzt. Anschließend kühlt man ab und engt i.V. ein. Man verrührt mit Wasser und saugt den ausgefallenen Niederschlag ab. Man erhält so nach Trocknung 10,5 g (83 % der Theorie) Cyanazauracil.

Analog werden hergestellt

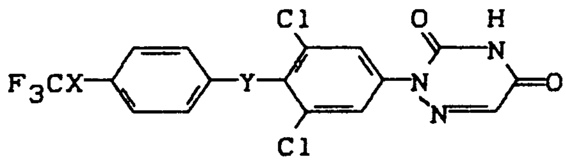

| | | |
|---|---|---|
| Bsp. 9 | X = O | Y = CHCN |
| Bsp. 10 | X = S | Y = CHCN |

V Beispiel für Verfahren 7

Beispiel 11

Ethyl-N-[[[cyano(4-(4'-trifluormethylthiophenyl)phenoxy)-hydrazinyliden]-methyl]-carbonyl]-carbonat

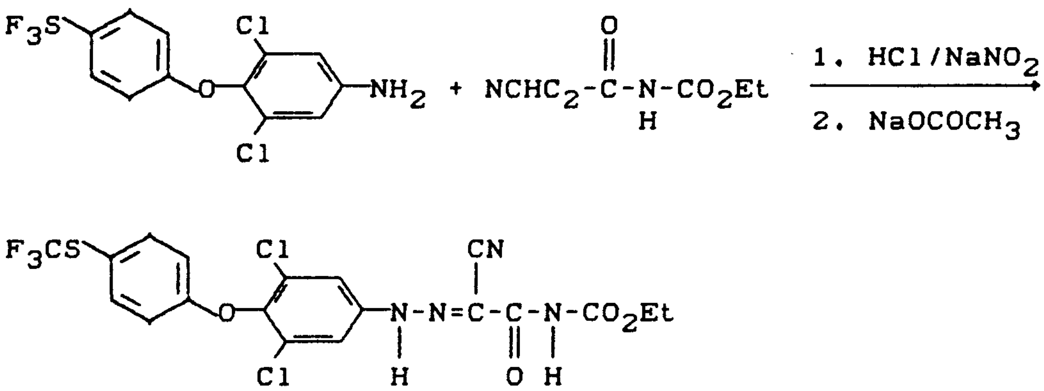


15,8 g (0,045 mol) Anilin werden in 10 ml konz. HCl und 100 ml Ethanol gelöst und bei 0-5 ° C mit einer Lösung aus 3,2 g (0.045 mol) Natriumnitrit in 30 ml Wasser tropfenweise versetzt. Man rührt bis zur klaren Lösung nach, gibt dann ein Gemisch aus 7,1 g (0,045 mol) Cyanacetylurethan und 11 g (0,13 mol) Natriumacetat zu und läßt 3 h bei 10 ° C nachrühren. Das Reaktionsgemisch wird i.V. eingeengt, mit Wasser verrührt und der Feststoff abgesaugt. Man erhält so 19 g (82 %) Produkt als feinkristallines gelbes Pulver.

Beispiel für Verfahren 8

Beispiel 12

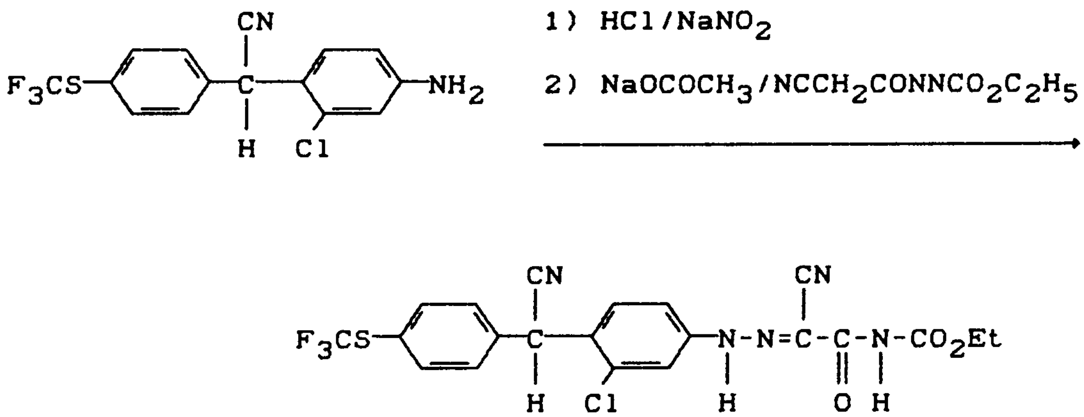


8,9 g (0,026 mol) Anilin werden in 5,6 ml konz. HCl und einem Gemisch aus 50 ml Eisessig und 50 ml Propionsäure gelöst und mit 1,8 g (0,026 mol) Natriumnitrit bei 0 ° C in 5 ml Wasser tropfenweise versetzt. Man rührt 30 min nach und tropft die so hergestellte Diazoniumsalzlösung in eine auf 0 ° C gekühlte Mischung aus 4 g (0,026 mol) Cyanacetylurethan und 5,3 g (0,065 mol) Natriumacetat in 40 ml Eisessig und 40 ml Propionsäure uns läßt 3 Stunden bei 10 ° C nachrühren. Das Reaktionsgemisch wird im Vakuum eingeengt, mit Wasser versetzt und der Feststoff abgesaugt. Man erhält so 3 g (76 %) der Hydrazinylverbindung als gelben Feststoff.

Analog werden hergestellt

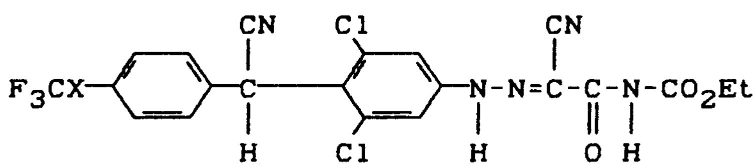

| | |
|---|---|
| Bsp. 13 | X = O |
| Bsp. 14 | X = S |

## Patentansprüche

**1.** Verbindungen der Formel (I)

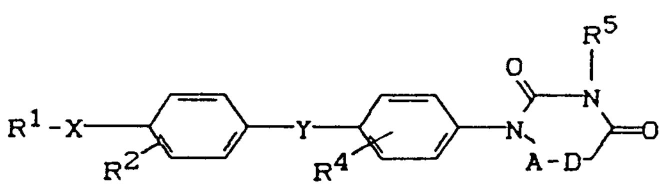

(I)

in welcher

A-D für -N = CH- oder

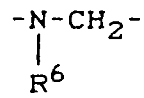

steht

X für O oder S, SO, $SO_2$ steht,

Y für O, S, CO,

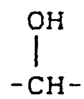 , 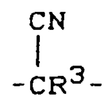

steht,

$R^1$ für $C_{1-4}$-Halogenalkyl steht,

$R^2$ für Wasserstoff, Halogen, $C_{1-4}$-Halogenalkyl steht,

$R^3$ für Wasserstoff, $C_{1-4}$-Alkyl steht,

$R^4$ für einen oder mehrere gleiche oder verschiedene Reste aus der Reihe Wasserstoff, Halogen, Halogenalkyl, $C_{1-4}$-Alkyl steht,

$R^5$ und $R^6$ unabhängig voneinander für Wasserstoff, $C_{1-4}$-Alkyl, Halogenalkyl, Aralkyl, Alkinyl stehen.

**2.** Verfahren zur Herstellung der neuen Verbindungen der Formel (I)

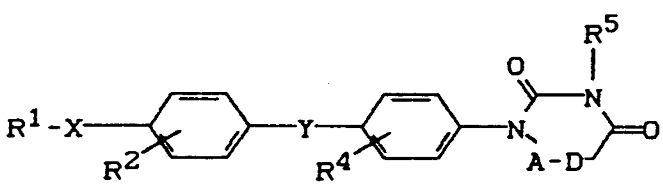 (I)

A-D für -N=CH- oder

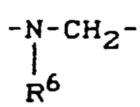

steht

X für O oder S, SO, $SO_2$ steht,

Y für O, S, CO,

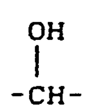 , 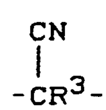

steht,

$R^1$ für $C_{1-4}$-Halogenalkyl steht,

$R^2$ für Wasserstoff, Halogen, $C_{1-4}$-Halogenalkyl steht,

$R^3$ für Wasserstoff, $C_{1-4}$-Alkyl steht,

$R^4$ für einen oder mehrere gleiche oder verschiedene Reste aus der Reihe Wasserstoff,

Halogen, Halogenalkyl, $C_{1-4}$-Alkyl steht,

$R^5$ und $R^6$ unabhängig voneinander für Wasserstoff, $C_{1-4}$-Alkyl, Halogenalkyl, Aralkyl, Alkinyl stehen,

dadurch gekennzeichnet, daß man in den Fällen in denen A-D für -N=CH- steht

a) für den Fall, daß $R^5$ für einen anderen Rest als Wasserstoff steht, Verbindungen der Formel (Ia)

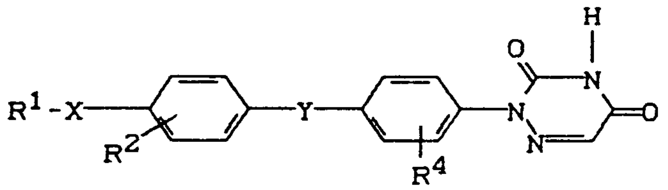

(Ia)

in welcher

Y, X, $R^1$, $R^2$, $R^3$, $R^4$ die in Anspruch (1) angegebene Bedeutung haben,

mit Verbindungen der Formel (II)

$R^5$-B (II)

in welcher

$R^5$ für gegebenenfalls substituiertes Alkyl, Alkenyl, Alkinyl, Aralkyl steht und

B für Halogen, $-O-SO_2$-Alkyl, $-O-SO_2$-Aryl, $-O-SO_2$-Halogenalkyl steht

umsetzt, oder

b) Verbindungen der Formel (III)

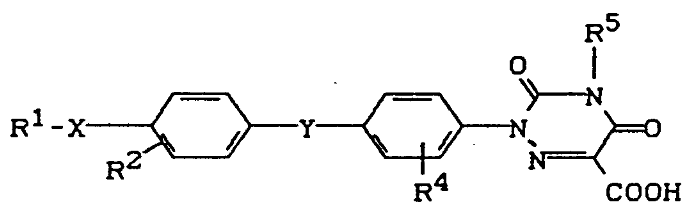

(III)

in welcher

Y, X, $R^1$, $R^2$, $R^3$, $R^4$, $R^5$ die in (1) angegebenen Bedeutungen haben,

durch Erhitzen decarboxyliert und ferner, daß man in den Fällen in denen A-D für

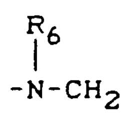

steht,

c) Verbindungen der Formel (Ib)

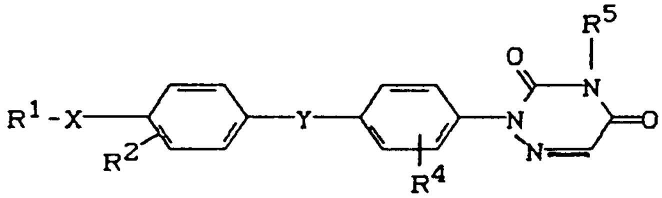

(Ib)

hydriert, oder

d) daß man Verbindungen der Formel

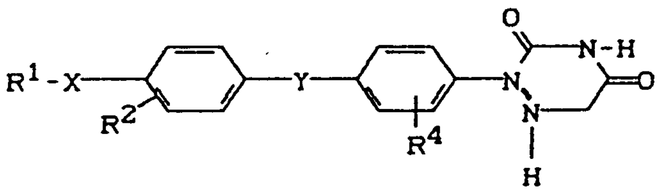

in welcher

Y, X, $R^1$, $R^2$, $R^3$, $R^4$ die oben angegebene Bedeutung haben

mit Verbindungen der Formel II

$R^5$-B (II)

in welcher

$R^5$ für gegebenenfalls substituiertes Alkyl, Alkenyl, Alkinyl, Aralkyl steht und

B für Halogen, -O-$SO_2$-Alkyl, -O-$SO_2$-Aryl, -O-$SO_2$-Halgoenalkyl steht

umsetzt, oder

e) daß man Verbindungen der Formel

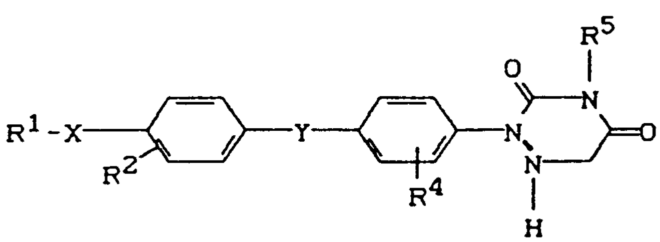

in welcher

Y, X, $R^1$, $R^2$, $R^4$, $R^5$ die oben angegebene Bedeutung haben, $R^5$ aber nicht für Wasserstoff steht,

mit Verbindungen der Formel VI

$R^6$-B (VI)

in welcher

$R^6$ und B die oben angegebene Bedeutung haben,

umsetzt.

**3.** Verbindungen der Formel (III)

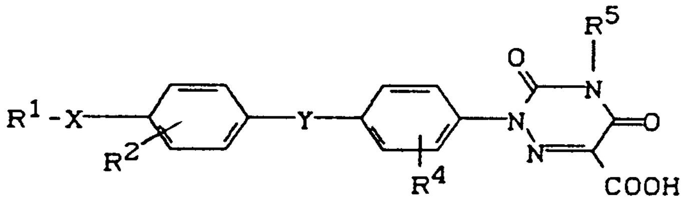

(III)

in welcher

Y, X, $R^1$, $R^2$, $R^3$, $R^4$, $R^5$ die in Anspruch 1 abgegebene Bedeutung haben.

**4.** Verfahren zur Herstellung der neuen Verbindungen der Formel (III) gemäß Anspruch 3, dadurch gekennzeichnet, daß man Verbindungen der Formel (IV)

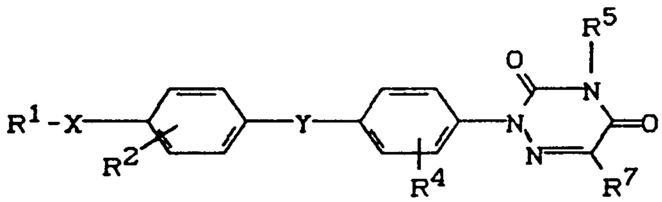

(IV)

in welcher

| | |
|---|---|
| Y, X, $R^1$, $R^2$, $R^3$, $R^4$, $R^5$ | die in Anspruch 1 angegebene Bedeutung haben, |
| $R^7$ | für CN oder den Rest -CO-N($R^5$)-COOR$^8$ steht, |
| $R^8$ | für Alkyl oder Aryl steht, |

in Gegenwart von wäßrigen Mineralsäuren erhitzt.

**5.** Verbindungen der Formel (IV)

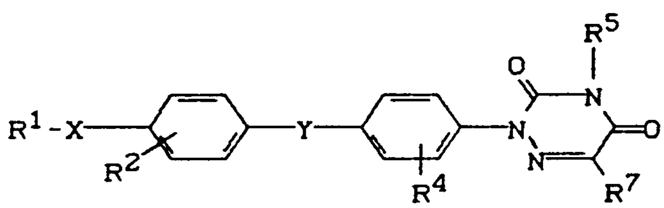

(IV)

in welcher

Y, X, $R^1$, $R^2$, $R^3$, $R^4$, $R^5$, $R^7$ die in Anspruch 4 angegebene Bedeutung haben.

**6.** Verfahren zur Herstellung der neuen Verbindungen der Formel (IV) gemäß Anspruch 5, dadurch gekennzeichnet, daß man Verbindungen der Formel (V)

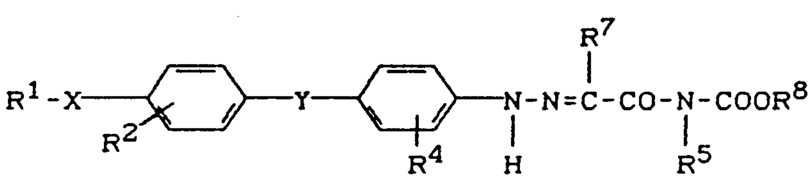

(V)

in welcher

Y, X, $R^1$, $R^2$, $R^3$, $R^4$, $R^5$, $R^7$, $R^8$ die in Anspruch 5 angegebene Bedeutung haben,

in Gegenwart von Basen erhitzt.

**7.** Neue Verbindungen der Formel (V)

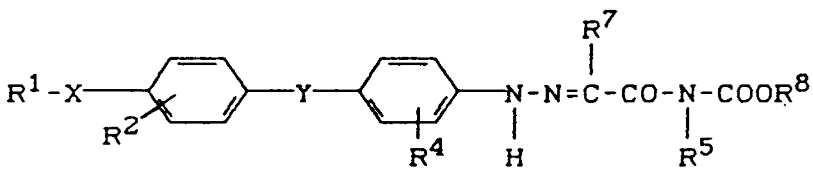

(V)

in welcher

Y, X, $R^1$, $R^2$, $R^3$, $R^4$, $R^5$, $R^7$, $R^8$ die in Anspruch 5 angegebene Bedeutung haben und $R^7$ zusätzlich für Wasserstoff stehen kann.

**8.** Verfahren zur Herstellung der neuen Verbindungen der Formel (V) gemäß Anspruch 7, dadurch gekennzeichnet, daß man Verbindungen der Formel (VI)

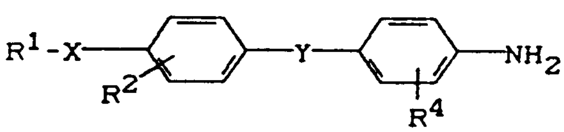

(VI)

in welcher

Y, X, $R^1$, $R^2$, $R^3$, $R^4$ die in Anspruch 1 angegebene Bedeutung haben,

in an sich bekannter Weise diazotiert und anschließend mit Verbindungen der Formel (VII)

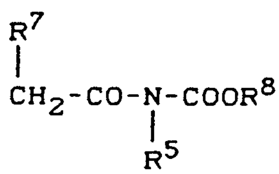

(VII)

in welcher

$R^3$, $R^7$, $R^8$ die in Anspruch 5 angegebene Bedeutung haben,

umsetzt.

**9.** Mittel gegen parasitische Protozoen, gekennzeichnet durch einen Gehalt an mindestens einem 1,2,4-Triazindion der Formel (I) gemäß Anspruch 1.

**10.** Verwendung von 1,2,4-Triazindionen der Formel (I) gemäß Anspruch 1 zur Herstellung von Mitteln gegen parasitische Protozoen.

Europäisches Patentamt

# EUROPÄISCHER RECHERCHENBERICHT

Nummer der Anmeldung

EP 91114977.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| A | EP - A - 0 383 285 (HOECHST) * Anspruch 1 * | 1,9,10 | C 07 D 253/065<br>A 61 K 31/53<br>A 01 N 43/707 |
| A | EP - A - 0 232 932 (JANSSEN) * Ansprüche 1,13; Formel 8 * | 1,6,7,9 | |
| A | DE - A - 2 722 537 (HOECHST) * Ansprüche 1,5 * | 1,9,10 | |
| A | DE - A - 3 531 919 (HOECHST) * Anspruch 1 * | 1 | |
| A | DE - A - 3 408 924 (HOECHST) * Ansprüche 1,5 * | 1,9,10 | |
| A | US - A - 3 883 528 (MYLARI) * Beispiele 27-41; Anspruch 1 * | 1 | RECHERCHIERTE SACHGEBIETE (Int Cl 5)<br>C 07 D 253/00 |
| A | US - A - 3 912 723 (MILLER) * Zusammenfassung; Beispiel II * | 1,9 | |
| A | DE - A - 2 423 972 (PFIZER) * Ansprüche 1,22; Beispiel 2 * | 1,9 | |
| A | DE - A - 3 904 593 (HOECHST) * Anspruch 1 * | 1,9,10 | |
| A | DE - A - 2 149 645 | 1,9,10 | |

Der vorliegende Recherchenbericht wurde für alle Patentansprüche erstellt.

| Recherchenort | Abschlußdatum der Recherche | Prüfer |
|---|---|---|
| WIEN | 09-12-1991 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

# EUROPÄISCHER RECHERCHENBERICHT

Nummer der Anmeldung

-2-

EP 91114977.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl ⁵) |
|---|---|---|---|
| | (PFIZER)<br>* Ansprüche 1,2; Beispiel 7 *<br>-- | | |
| A | CHEMICAL ABSTRACTS, Band 91, Nr. 19, 5. November 1979, Columbus, Ohio, USA<br>ROESNER, MANFRED et al. "Substituted 2-phenyl-1,2,4--triazine-3,5(2H,4H)-diones and coccidiostatic agents containing them"<br>Seite 645, Spalte 1, Zusammenfassung-Nr. 157 771d<br>& S. African 78 02,817<br>---- | 1,6,7 | |

RECHERCHIERTE SACHGEBIETE (Int Cl ⁵)

Der vorliegende Recherchenbericht wurde für alle Patentansprüche erstellt.

| Recherchenort | Abschlußdatum der Recherche | Prüfer |
|---|---|---|
| WIEN | 09-12-1991 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 62